# EUROPEAN PATENT APPLICATION

(11) **EP 1 658 814 A2**
(43) Date of publication of application: **24.05.2006**
(21) Application number: 05257060.3
(22) Date of filing: 16.11.2005
(51) Int. Cl.: A61B 17/12, A61K 45/06, A61M 25/10, A61K 9/00, A61B 6/00, A61B 8/12

(54) **Methods for evaluating pathologies or therapeutics in a rat carotid artery**

(30) Priority: 17.11.2004 US 991188
(71) Applicant: Cordis Corporation, Miami Lakes, FL 33014 (US)
(72) Inventor: Argentieri, Dennis C., Milford, NJ 08848 (US); Falotico, Robert, Belle Mead, NJ 08502 (US); Parry, Tom Jay, Hellertown, PA 18055 (US); Zhao, Jonathon Z., Belle Mead, NJ 08502 (US)
(74) Representative: Belcher, Simon James

(57) **Abstract**

A methodology for evaluating the efficacy of locally applied antiproliferatives and/or other therapeutic agents on neointimal hyperplasia, as well as immunohistochemical and vascular cell cycle changes, in balloon-injured rat carotid arteries may be utilized for determining the efficacy of treatments in humans.

In one aspect, a method for evaluating pathologies in a rat carotid artery is described. The method comprises: endovascularly creating an injury in at least one location within the carotid artery of a rat; and evaluating the pathologies associated with the injury.

## Description

The present invention relates to methods and systems for evaluating mammalian arteries, and more particularly to methods and systems for evaluating pathologies and therapeutics in rat carotid arteries.

A number of model systems have been developed to evaluate the safety and efficacy of therapeutic agents in reducing restenosis following vascular injury. Evaluation of antirestenotic therapies and processes involved in arterial injury may be conducted in a variety of arteries with different morphologies. Muscular arteries (e.g. coronary arteries) have layers of smooth muscle cells that are defined by internal and external elastic lamina. Conduit arteries of various animals, for example, the carotid artery, the iliac arteries and the aorta, have been commonly utilized in the assessment of potential anti-restenosis therapies.

The typical animal model is utilized to determine the safety and efficacy of therapeutic agents as well as the means of delivery. The models are designed to evaluate a predetermined set of parameters that are limited in scope. Accordingly, there is needed a model having a broader scope. In addition, there exists a need for a methodology of evaluating perivascular delivery of therapeutic agents as well as devices for perivascular delivery.

The present invention overcomes the limitations associated with currently utilized animal models as briefly described above.

The present embodiment includes the use of an elastic artery model system, the rat carotid artery injury model, which may be optimized to study the pathophysiologic processes related to arterial injury and to evaluate the effects of locally or systemically applied therapies that impact those pathophysiologic processes.

In accordance with one aspect, the present invention is directed to a method for evaluating pathologies in a rat carotid artery. The method comprises the steps of creating an endovascular injury in at least one location within the carotid artery of a rat and evaluating the pathologies associated with the injury.

In accordance with another aspect, the present invention is directed to a method for evaluating therapeutics in a rat carotid artery. The method comprises endovascularly creating an injury in at least one location within the carotid artery of a rat, treating the injured artery, and evaluating the pathologies associated with the treated injured artery and the efficacy of the treatment (local or systemic).

The present invention is directed to a methodology or model for evaluating the efficacy of locally applied antiproliferatives and/or other therapeutic agents on neointimal hyperplasia, as well as immunohistochemical and vascular cell cycle changes in balloon-injured rat carotid arteries. As described herein, a number of models have been developed to evaluate the efficacy of therapeutic agents in reducing restenosis following vascular injury. The present invention, however, is directed to a model system for the evaluation of locally applied or locally delivered agents for their ability to reduce neointimal hyperplasia and affect vascular cell cycle phases following balloon-injury in the rat carotid artery. In addition, the present invention extends to multiple, potential rat disease models, induced and spontaneous, that exist, including diabetes mellitus (Types I and II), atherosclerosis, immunologically altered, transgenics and knock-outs. Further, the application of agents locally to influence lesion phenotypes (e.g. exacerbation of inflammation, upregulation of smooth muscle cell proliferation, application of genes affecting these processes) may enhance the study of vascular wall responses to injury and inflammation. One embodiment is to influence the nature of the carotid artery lesion by a combination of injury, dyslipidemia (e.g. high fat/cholesterol diet) and local inflammation in such a way that a vulnerable plaque phenotype (thin fibrous cap, lipid rich necrotic core, presence of foam cells and macrophages, presence of T cells and potential for ulceration/erosion) is formed.
Embodiments of the invention will now be described by way of example with reference to the accompanying drawings, in which:
Figure 1 is a diagrammatic representation of a perivascular therapeutic agent delivery vehicle in accordance with the present invention.
Figure 2 is a graphical representation of the effects of perivascular application of sirolimus and paclitaxel on neointimal hyperplasia in an injured rat carotid artery in accordance with the present invention.
Figure 3 is a series of slides illustrating neointimal formation in rat carotid arteries undergoing treatment in accordance with the present invention.
Figures 4 and 5 are graphical representations of the effects of local sirolimus and paclitaxel application on cell proliferation and apoptosis within the injured rat carotid artery in accordance with the present invention.
Figures 6 and 7 are graphical representations of the effects of perivascular applications of sirolimus and paclitaxel on vascular wall cell cycle and the number of apoptotic nuclei within the injured rat carotid artery in accordance with the present invention.

The present invention is directed to a methodology or model for the evaluation of the effects of perivascularly-applied medical devices around rat carotid arteries either exposed or not exposed to endovascular injury. This methodology or model may serve as a means for the evaluation of therapeutic agents for the treatment of vascular diseases, including restenosis and vulnerable plaque, and may also serve to model safety and efficacy testing of any number of endovascularly applied therapeutic agents in the context of normal or atherosclerotic arteries. Essentially, the present invention makes use of a balloon catheter to produce an endovascular injury. The endovascular injury may be induced via other means, including diet, perivascular injection/administration of proinflammatory agents and/or systemic drug delivery. In addition, the application of standard endpoint evaluation, intravital microscopy, including angiography, intravascular ultrasound imaging, histological, molecular and cellular endpoints, may be utilized in conjunction with the approaches described herein.

The methodology of the present invention comprises a number of procedures or steps. The methodology should be understood not to be limited to the particular devices and therapeutic agents and/or other drugs described herein.

In accordance with one exemplary embodiment, the present invention is directed to a methodology or model for evaluating the efficacy of locally applied antiproliferatives and/or other therapeutic agents on neointimal hyperplasia, as well as immunohistochemical and vascular cell cycle changes in balloon-injured rat carotid arteries. As described herein, a number of models have been developed to evaluate the efficacy of therapeutic agents in reducing restenosis following vascular injury. The present invention, however, is directed to a model system for the evaluation of locally applied or locally delivered agents for their ability to reduce neointimal hyperplasia and affect vascular cell cycle phases following balloon-injury in the rat carotid artery. In addition, the present invention extends to multiple, potential rat disease models, induced and spontaneous, that exist, including diabetes mellitus (Types I and II), atherosclerosis, immunologically altered, transgenics and knock-outs.

The model or methodology comprises essentially three aspects; namely, therapeutic agent delivery vehicle preparation, surgical intervention, including injury induction and vehicle introduction, and evaluation. Although there are a wide variety of means for introducing the therapeutic agent or agents, in the exemplary embodiment described herein, the agents are delivered utilizing pluronic gels. Many pluronic gels are thermally sensitive polymers that are liquids at certain temperatures, for example, low temperatures, and form highly viscous polymers at warmer temperatures, for example, body temperature. These polymers have been utilized as local delivery depots for antiproliferative drugs upon direct injection into tumors (Amiji et al., Pharm Dev Technol. 2002 May; 7(2):195-202).

The present invention embodies the use of such gels, with or without encasement, for the local delivery of drugs or agents to the external or adventitial surface of injured blood vessels to evaluate the drug's effect on neointimal formation and other cellular/molecular end points. The present invention also combines the use of a local drug delivery system with methodologies to evaluate the effects of locally applied drugs on vascular morphology, for example, the formation of neointimal, vascular wall cell cycle changes, changed in cell types in the vessel wall, and gene expression (mRNA and protein) following vascular-injury.

It is important to note that any number of pluronic gels may be formulated. In the exemplary embodiment set forth below, the procedures for formulating Pluronic F127 for the suspension of therapeutic agents or drugs, including sirolimus and paclitaxel, for local application around a blood vessel are detailed. In the exemplary embodiment, the pluronic gel is formulated at twenty-five percent by weight in sterile water. The concentration of the pluronic gel and composition of the solvent may be modified to affect the delivery characteristics of a particular drug or agent to the vessel wall. In addition, the polymer may be substituted to accommodate desired delivery specifications.

Pluronic F-127 may be obtained from a number of sources, including from Sigma (P-2443, 250 g, Lot121K0070, BASF). Once the Pluronic F-127 is obtained, sterile deionized water is prepared and chilled, for example, on ice. Ten grams of the Pluronic F-127 is then mixed with the sterile, deionized water for a final concentration of 10 g/40 mL. It should be noted that upon initiation of mixing, the poloxamer and water are inherently poorly miscible. However, constant stirring utilizing a sterile plastic pipette, for example, a 1 ml tissue culture pipette, allows for good mixing. The Pluronic F-127 slowly dissolves in the chilled water with constant stirring to yield a clear but foamy solution. The Pluronic F-127 solution is then refrigerated for a period ranging from about 6 to 12 hours to allow for defoaming. The defoamed solution is now ready for perivascular implantation.

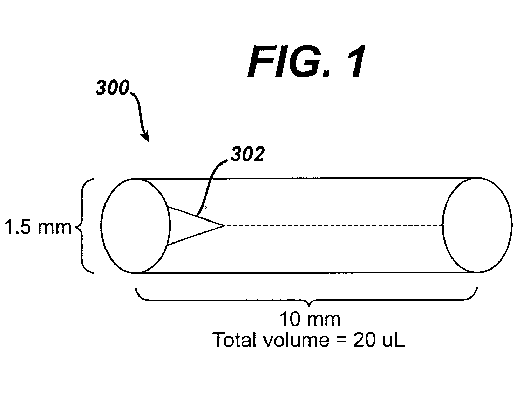

The delivery vehicle may comprise any suitable device. In the exemplary embodiment, the delivery vehicle comprises silastic tubing as illustrated in Figure 1. The silastic tubing 300 is about 10 mm long with an outside diameter of about 1.5 mm. The total luminal volume, based upon empirical measurement, is about 20 µL. It is important to note that the silastic tubing 300 may comprise any suitable length. The length of the tubing is determined by the amount of artery resected. The silastic tubing may comprise a notch 302 on one end to facilitate placement thereof over the carotid artery. The silastic tubing is then autoclaved prior to surgery. The silastic tubing is filled with about 20 µL of the pluronic gel comprising a suspension, solution or combination suspension and solution of test agent. The pluronic gel formulation to be encased within the silastic tubing is dispersed into the silastic tubing utilizing a 20 µL adjustable pipette with a sterile tip. The pluronic vehicle or pluronic drug solution remains on ice until immediately before pipetting and dispensing into the silastic tubing.

The next step in the process comprises the surgical procedure. The rat is anesthetized prior to surgical intervention. The rat may be anesthetized utilizing any number of anesthetizing agents, both inhalational and/or injectable. In the exemplary embodiment, the rat, typically weighing between 250 and 450 mg, is anesthetized with a mixture of ketamine/xylazine/acepromazine (50/10/1 mg/kg). Once the rat is aneshetized, the central neck region is shaved. The neck is then disinfected by swabbing first with 70 percent ethanol or isopropanol, then with povidone iodine and then with 70 percent ethanol. The rat is then wrapped from the chest with a sterile drape and then placed on a warming pad set to a temperature of about 37 degrees C. A midline incision is then made to expose the underlying glands and musculature. The salivary complex and musculature overlying the external carotid artery is bluntly dissected away to expose the left external carotid artery. Blood flow in the external carotid is controlled by proximal (a removable loop) and distal (permanent) ligature. Upon control of blood flow, an arteriotomy is created through which a 2 F Fogarty embolectomy catheter is introduced. The catheter is advanced proximally through the external carotid into the common carotid artery to the first mark (approximately 3 cm) on the catheter shaft. The balloon is inflated using a saline-filled threaded gas-tight syringe, available under the trade name Hamilton, and then gently pulled distally through the common carotid artery using a slight twisting motion. Once the common carotid artery is balloon-injured, the balloon is deflated and the balloon injury procedure is repeated for a total of three times. Following balloon injury, the catheter is withdrawn through the arteriotomy and bleeding is controlled by permanently ligating the proximal external carotid artery ligature. Confirmation of common and internal carotid artery pulsation is made.

Following balloon injury, the left common carotid artery within the neck region is then gently dissected away from connective and nerve tissue. A silastic tube, as described above, is then filled with either Pluronic F-127 or a suspension of test agent in Pluronic F-127. The silastic tube, with a slit along its longitudinal axis, is then gently introduced around the adventitial side of the exposed common carotid artery, thereby initiating perivascular drug or Pluronic F-127 delivery. The overlying musculature and connective tissue is then repaired with sutures and the skin wound closed using stainless steel surgical sutures. The closed wound is re-swabbed with povidone iodine and 5 mL of sterile saline is given subcutaneously for fluid replacement. Rats are allowed to recover for up to twenty-one days depending upon the desired endpoint.

For assessment of neointimal hyperplasia (fourteen to twenty-one days, depending upon desired time point) or immunohistochemisry (one to twenty-one days, depending on desired marker expression), rats are euthanized according to AVMA 2000 recommendations by overexposure to carbon dioxide. Upon cessation of breathing, the chest of the rat is opened to expose the heart. The right atrium is cut and the left atrium is perfused with normal saline to exsanguinate, which is then followed by perfusion with neutral buffered formalin for *in situ* fixation. The treated region of the injured common carotid artery is then harvested and the perivascular deployed silastic tube is removed. The vessel is then stored in neutral buffered formalin for subsequent histologic preparation and histomorphometric analysis.

Fixed vessels are then embedded in paraffin and sectioned at three levels. Vessel sections are mounted on coated glass slides and stained with either hematoxylin/eosin or Verhoff van Geisson elastic stain. Lumenal, neointimal and medial areas are then assessed using computerized morphometric analysis programs calibrated using a stage micrometer. Cell proliferation, apoptosis or expression of other cell markers is obtainable using this method. In this exemplary embodiment, cell proliferation and apoptosis are evaluated using anti-Proliferating cell Nuclear Antigen (PCNA) and Terminal deoxynucleotide transferase-mediated dUTP nick-End labeling (TUNEL) immunohistochemistry.

For assessment of vascular cell cycle, vessels are preferably harvested between one to ten days (more preferably three or seven days) from euthanized, non-perfused rats (see above). Vessles are then thoroughly minced using crossed scalpels and placed in a DMSO/citrate/sucrose cryoprotection solution. The mincate is then snap-frozen and stored at 80 degrees C for subsequent anaylsis. Upon analysis, mincates are treated with a trypsin/detergent solution which effectively extrudes cell nuclei from the mincate. The digestion is terminated, the suspension centrifuged, and the resulting nuclear suspension is treated with propidium iodide, forming a fluorescent complex with nuclear DNA. The propidium iodide-stained nuclear suspension is then drawn into a flow cytometer gated to eliminate debris and doublet nuclei for quantitation of fluorescence intensity. The resulting fluorescence is proportional to the amount of DNA in each nucleus. Approximately 12,000-20,000 events are obtained for subsequent analysis of cell cycle histograms. Cell cycle analysis is perfomed following acquisition of the DNA histogram using the MODFIT cell cycle analysis algorithm. Alternately, other cell markers may be analyzed using flow cytometry from whole cell preparations of minced vessels. This may include, but are not limited to, smooth muscle α-actin, PCNA, BrdU, Mac-1, interins, cell surface receptors, etc.

Mincates from treated and injured or non-injured vessel walls may be subjected to extraction of RNA and subsequent gene expression using quantitative RT-PCR and/or gene chip analysis.

Gene expression may also be evaluated in cryo-sections of snap-frozen treated/injured or non-injured blood vessels using in situ hybridization followed by film autoradiography, emulsion autoradiography fluorescence (FISH).

Protein expression may be evaluated by histologic (immunohistochemistry) or from protein extracts of minced treated/injured or non-injured carotids using ELISA's or other assays.

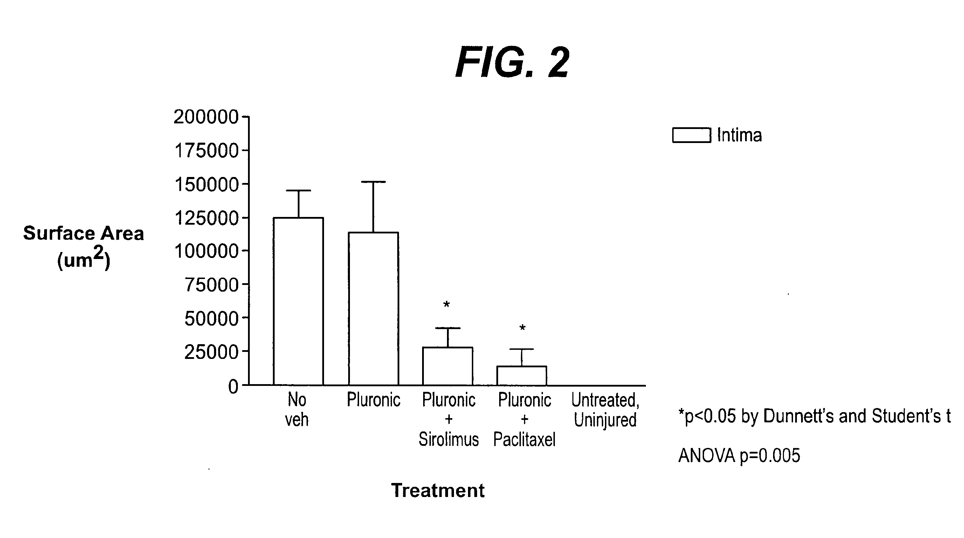

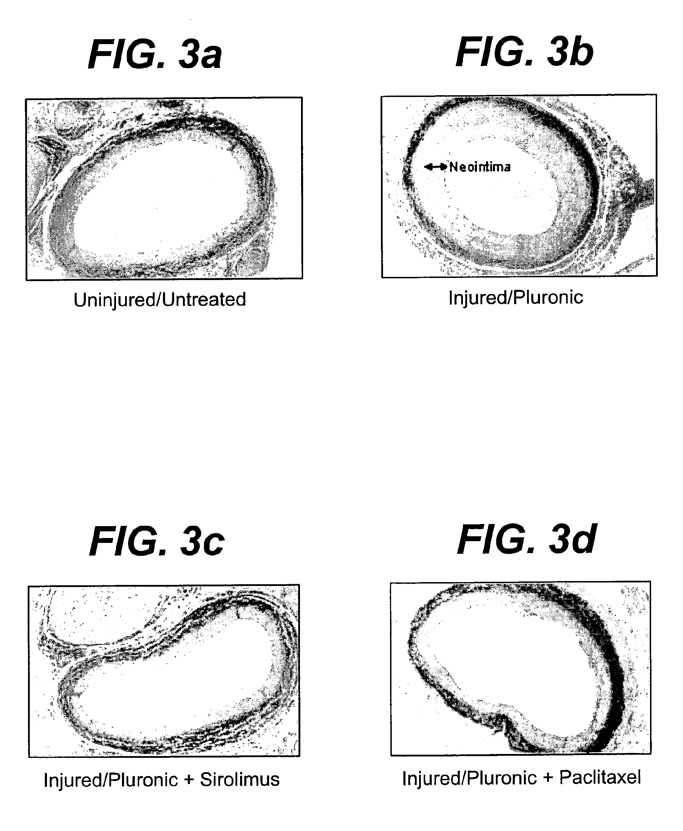

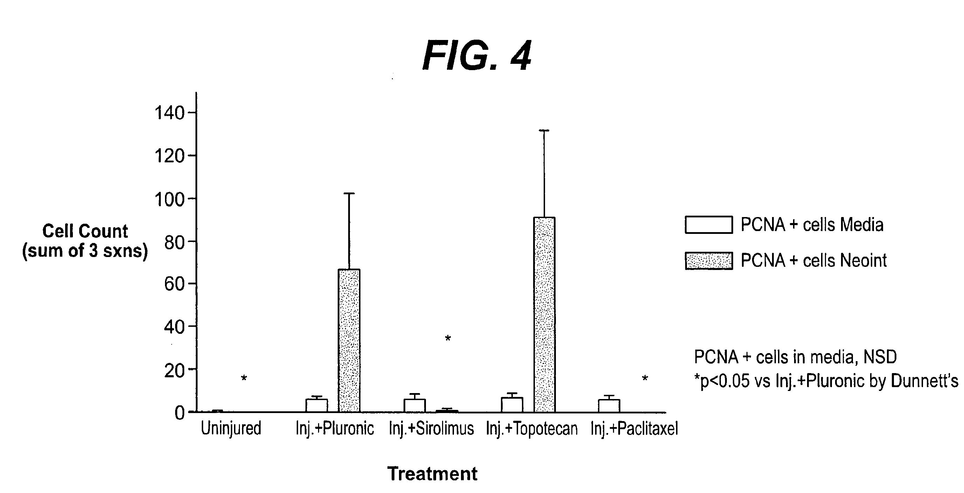

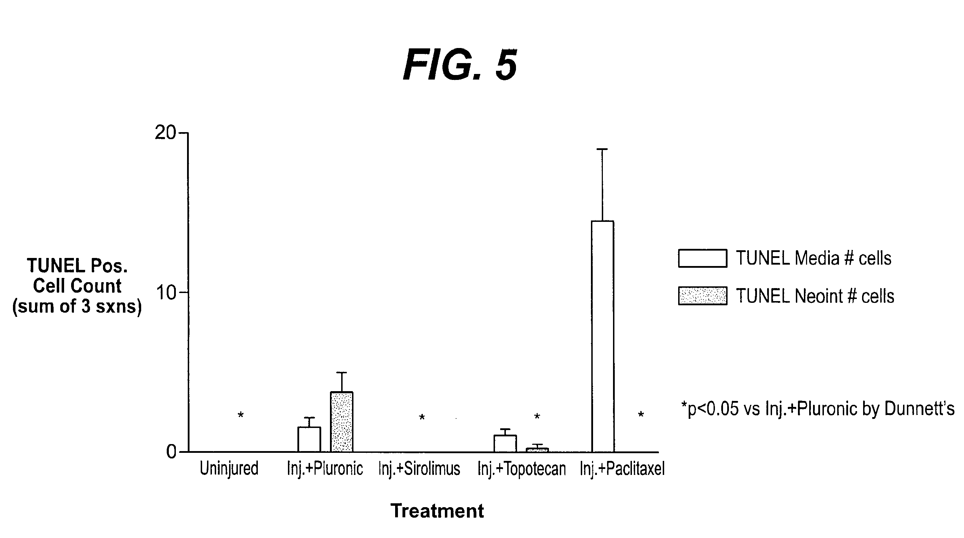

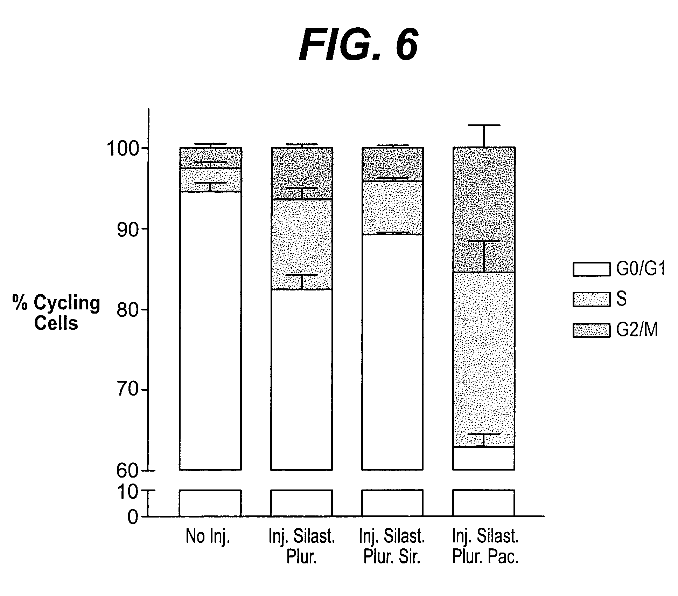

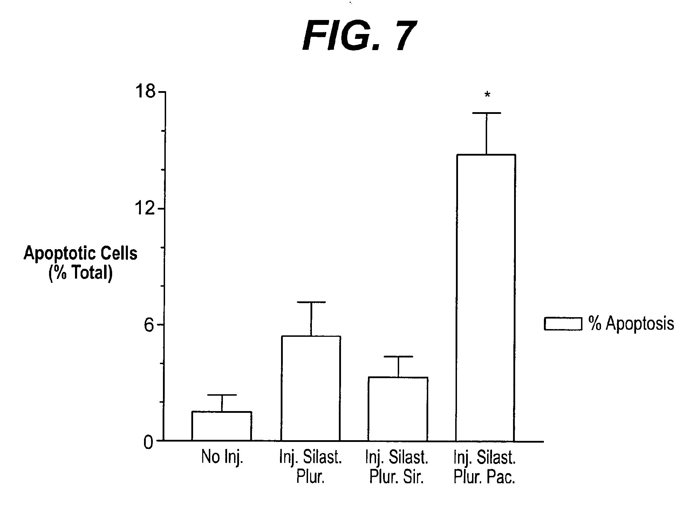

Relevant data utilizing the drug delivery system in the context of neointimal formation, cell proliferation, apoptosis and call cycle changes are illustrated in Figures 2-7. Figure 2 illustrates the effects of perivascular application of sirolimus and paclitaxel on neointimal hyperplasia in the injured rat carotid artery. As is shown in Figure 2, perivascularly applied sirolimus and paclitaxel significantly reduced neointimal fomation in the injured rat carotid artery. Data in Figure 2 are presented as mean +/- SEM. Figure 3 contains slide images of rat carotid arteries. In the first slide, there is shown an uninjured and untreated carotid artery. In the next slide, there is shown an injured carotid artery exposed to just the pluronic gel. As may be seen from this slide, the injury caused the formation of a substantial neointima. In the next slide, there is shown an injured carotid artery treated with sirolimus in combination with the pluronic gel. In the exemplary embodiment 200 µg of sirolimus was added to the pluronic gel. The result of the perivascular administration of sirolimus was a substantial reduction in neointima formation. In the final slide, there is shown an injured carotid artery treated with paclitaxel in combination with the pluronic gel. In the exemplary embodiment 200 µg of paclitaxel was added to the pluronic gel. As was the case with sirolimus, paclitaxel administered perivascularly resulted in a substantial-reduction of neointima formation. Figures 4 and 5 are graphical representations of Cell Count and TUNEL Pos. Cell Count for vascular treatment protocols. Essentially, these two figures illustrate the effects of local sirolimus and paclitaxel delivery on all proliferation (upper panel PCNA immunohistochemistry) and apoptosis (lower panel TUNELimmunohistochemistry) within the injured rat carotid artery seven days post injury. Note that proliferation (PCNA labeling) increases in the neointima seven days after injury. Sirolimus and paclitaxel, in concentrations of 200 µg in pluronic gel suspension, significantly reduce neointimal proliferation. Topotecan delivered at a 200 µg perivascular dose has substantially no effect on neointimal proliferation. Data are presented as mean +/- SEM. Figures 6 and 7 are graphical representations of the effects of perivascular application of sirolimus and paclitaxel (200 µg in pluronic gel suspension) on vascular wall cell cycle (upper panel) and the number of apoptotic nuclei (lower panel) within the injured rat carotid artery as assessed by flow cytometry seven days post injury. It is important to note that injury increases the percentage of nuclei in the S and G2/M phases of the cell cycle while decreasing those in the G0/G1 phase. Consistant with its proported mechanisim of action, sirolimus restores the percentage nuclei in the G0/G1 phases while reducing those in the S and G2/M phases. The mitotic arresting agent paclitaxel increases the percentage of cells in G0/G1. In the lower panel, paclitaxel is shown to increase the total percentage of apoptotic cells in the injured vessel wall. Data are presented as mean +/- SEM.

A partial list of therapeutic and pharmaceutical agents that may be utilized alone or in conjunction with implantable medical devices include antiproliferative/antimitotic agents including natural products such as vinca alkaloids (i.e. vinblastine, vincristine, and vinorelbine), paclitaxel, epidipodophyllotoxins (i.e. etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiplatelet agents such as G(GP) 11_{b}/111ₐ inhibitors and vitronectin receptor antagonists; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nitrosoureas (carmustine (BCNU) and analogs, streptozocin), triazenes - dacarbazinine (DTIC); antiproliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine {cladribine}); platinum coordination complexes (cisplatin, carboplatin), procarbazine, hydroxyurea, mitotane, aminoglutethimide; hormones (i.e. estrogen); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); anti-inflammatory: such as adrenocortical steroids (cortisol, cortisone, fludrocortisone, prednisone, prednisolone, 6a-methylprednisolone, triamcinolone, betamethasone, and dexamethasone), non-steroidal agents (salicylic acid derivatives i.e. aspirin; para-aminophenol derivatives i.e. acetaminophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); angiogenic agents: vascular endothelial growth factor (VEGF), fibroblast growth factor (FGF); angiotensin receptor blockers; nitric oxide donors; oligionucleotides and combinations thereof; cell cycle inhibitors, mTOR inhibitors, and growth factor receptor signal transduction kinase inhibitors; retenoids; cyclin/CDK inhibitors; HMG co-enzyme reductase inhibitors (statins); and protease inhibitors.

Although shown and described is what is believed to be the most practical and preferred embodiments, it is apparent that departures from specific designs and methods described and shown will suggest themselves to those skilled in the art and may be used without departing from the scope of the invention. The present invention is not restricted to the particular constructions described and illustrated, but should be constructed to cohere with all modifications that may fall within the scope of the appended claims.

## Claims

1. A method for evaluating pathologies in a rat carotid artery comprising:
endovascularly creating an injury in at least one location within the carotid artery of a rat; and
evaluating the pathologies associated with the injury.

2. A method for evaluating therapeutics in a rat carotid artery comprising:
endovascularly creating an injury in at least one location within the carotid artery of a rat;
treating the injured artery; and
evaluating the pathologies associated with the treated injured artery and the efficacy of the treatment.
